# EUROPEAN PATENT APPLICATION

(11) **EP 3 725 233 A1**
(43) Date of publication of application: **21.10.2020**
(21) Application number: 18888458.9
(22) Date of filing: 30.01.2018
(51) Int. Cl.: A61B 8/06, A61B 8/08, A61B 8/00

(54) **ULTRASONIC DIAGNOSIS APPARATUS AND CONTROL METHOD THEREFOR**

(30) Priority: 11.12.2017 KR 20170168920
(71) Applicant: Samsung Medison Co., Ltd., Hongcheon-gun, Gangwon-do, 25108 (KR)
(72) Inventor: YIM, Yu Ri, Seoul 07247 (KR); BYUN, Mi Ae, Seongnam-si, Gyeonggi-do 13260 (KR); YANG, Eun Ho, Seoul 01774 (KR)
(74) Representative: Arnold & Siedsma
(86) International application number: PCT/KR2018/001278
(87) International publication number: WO 2019/117395

(57) **Abstract**

Embodiments of the present disclosure relate to an ultrasonic diagnostic apparatus and a control method thereof to perform a leg vein examination more easily, and a control method thereof.

An ultrasonic diagnostic apparatus includes: an ultrasonic probe configured to acquire a color Doppler image; a controller configured to determine a presence or absence of backflow of blood and a time of backflow based on the acquired color Doppler image; and a display configured to display an examination result with the color Doppler image.

## Description

### Technical Field

Embodiments of the present disclosure relate to an ultrasonic diagnostic apparatus and a control method thereof, and more particularly, to an ultrasonic diagnostic apparatus that can perform a leg vein examination more easily, and a control method thereof.

### Background Art

An ultrasonic diagnostic apparatus is an apparatus which irradiates ultrasonic waves toward a target point inside an object from the surface of the object, and receives echo ultrasonic waves reflected from the target point so as to non-invasively obtain a tomographic image of soft tissue of the object or an image of blood flow of the object.

The ultrasonic diagnostic apparatus has a compact size and is cheaper, compared to other medical imaging apparatuses, such as an X-ray imaging apparatus, a Computerized Tomography (CT) scanner, and a Magnetic Resonance Image (MRI) apparatus, and is widely used because it can display diagnosis images in real time.

The ultrasonic diagnostic apparatus includes a probe to transmit ultrasonic waves to the object and to receive echo ultrasonic waves reflected from the object, in order to obtain an ultrasonic image of the object.

In the case of performing a vein examination of the lower extremity through a conventional ultrasonic diagnostic apparatus, there is an inconvenience in manually entering each mode when entering a mode for examination. In addition, there is the inconvenience of fixing the probe to the vascular region with one hand, performing system operation with the other hand, and squeezing and releasing the vascular region of the patient in the course of the examination.

### Disclosure

### Technical Problem

Therefore, it is an aspect of the present disclosure to provide an ultrasonic diagnostic apparatus for performing an inspection protocol for locating a sample volume of a pulse wave after detecting the presence or absence of backflow of blood flow.

### Technical Solution

In accordance with an aspect of the present disclosure, an ultrasonic diagnostic apparatus may comprise an ultrasonic probe configured to acquire a color Doppler image; a controller configured to determine a presence or absence of backflow of blood and a time of backflow based on the acquired color Doppler image; and a display configured to display an examination result with the color Doppler image.

The examination result may include at least one of the presence or absence of backflow of blood and the time of backflow.

The controller may automatically set a sample volume of a pulse wave in the color Doppler image based on the color Doppler image, and analyze a blood flow spectrum of the sample volume based on a blood flow information acquired from the ultrasonic probe.

The controller may calculate a blood flow velocity including at least one of an average velocity, a maximum velocity and a minimum velocity by analyzing the blow flow spectrum of the sample volume.

The display may display a result of the blood flow spectrum analysis of the sample volume.

The result of the blood flow spectrum analysis may include at least one of the presence or absence of backflow of blood, the time of backflow, amount of blood flow, blood flow rate, and blood flow direction.

The controller may determine a diagnostic grade for the time of backflow, and the display may display the determined diagnostic grade.

The ultrasonic diagnostic apparatus may include an input configured to receive an execution command for a color Doppler mode from a user in order to analyze the backflow of blood.

The display may display information about a subject's body parts that have been observed and results of an analysis on the subject's body parts.

The time of blood backflow may obtain based on the time of release after squeezing the body part of the subject.

In accordance with another aspect of the present disclosure, a control method of an ultrasonic diagnostic apparatus may comprise: acquiring a color Dopplier image from an ultrasonic probe; determining a presence or absence of backflow of blood and a time of backflow based on the acquired color Doppler image; and displaying an examination result with the color Doppler image.

The method may further comprise: automatically setting a sample volume of a pulse wave in the color Doppler image based on the color Doppler image; analyzing a blood flow spectrum of the sample volume based on a blood flow information acquired from the ultrasonic probe; and displaying a result of the blood flow spectrum analysis.

Analyzing the blood flow spectrum may comprise: calculating a blood flow velocity including at least one of an average velocity, a maximum velocity and a minimum velocity by analyzing the blow flow spectrum of the sample volume.

The method may further comprise: determining a diagnostic grade for the time of backflow, and displaying the determined diagnostic grade.

The time of blood backflow may include the time of blood backflow obtained based on the time of release after squeezing the body part of the subject.

### Advantageous Effects

According to an ultrasonic diagnostic apparatus and control method according to the disclosed embodiment, the following effects can be expected.

First, by automating a protocol for inspection, it is possible to reduce an inspection phase and fatigue of the examiner and to perform an accurate inspection.

In addition, by automatically performing backflow detection and waveform analysis of the pulse wave, it is possible to shorten the inspection time and improve the inspection step.

In addition, in the prior art, most of the tests were performed in a standing manner, which increased the burden on the patient during a long-term test, but a test time can be shortened to increase patient satisfaction.

### Description of Drawings

FIG. 1 is a view illustrating an appearance of an ultrasonic diagnostic apparatus according to an embodiment;
FIG. 2 is a view illustrating an appearance of an ultrasonic probe including a 1-Dimensional (ID) array transducer according to an embodiment;
FIG. 3 is a view illustrating an appearance of the ultrasonic probe including a 2-Dimensional (2D) array transducer according to an embodiment;
FIG. 4 is a view illustrating a relationship between the ultrasonic probe and a mian body according to an embodiment;
FIG. 5 is a control block diagram of the ultrasonic diagnostic apparatus according to an embodiment;
FIG. 6 is a flowchart illustrating a control method of the ultrasonic diagnostic apparatus according to an embodiment;
FIG. 7 is a diagram illustrating a workflow according to the control process of FIG. 6.
FIG. 8 is a diagram illustrating an example in which the diagnostic grade for the time of backflow is displayed on the first and second test result display screens.
FIG. 9 is a diagram illustrating an example in which a body marker is displayed on the first and second test result display screens.

### Mode for Invention

Configurations illustrated in the embodiments and the drawings described in the present specification are only the preferred embodiments of the present disclosure, and thus it is to be understood that various modified examples, which may replace the embodiments and the drawings described in the present specification, are possible when filing the present application.

Also, like reference numerals or symbols denoted in the drawings of the present specification represent members or components that perform substantially the same functions.

The terms used in the present specification are used to describe the embodiments of the present disclosure. Accordingly, it should be apparent to those skilled in the art that the following description of exemplary embodiments of the present disclosure is provided for illustrative purposes only and not for the purpose of limiting the disclosure as defined by the appended claims and their equivalents. It is to be understood that the singular forms "a," "an," and "the" include plural referents unless the context clearly dictates otherwise. It will be understood that when the terms "includes," "comprises," "including," and/or "comprising," when used in this specification, specify the presence of stated features, figures, steps, components, or combinations thereof, but do not preclude the presence or addition of one or more other features, figures, steps, components, members, or combinations thereof.

It will be understood that, although the terms first, second, etc. may be used herein to describe various components, these components should not be limited by these terms. These terms are only used to distinguish one component from another. For example, a first component could be termed a second component, and, similarly, a second component could be termed a first component, without departing from the scope of the present disclosure.

As used herein, the term "and/or" includes any and all combinations of one or more of associated listed items.

Also, the terms "front," "rear," "upper," and "lower," when used in this description, are defined based on the drawings, and the shapes and locations of the corresponding components are not limited by the terms.

Hereinafter, the embodiments of the present disclosure will be described in detail with reference to the accompanying drawings.

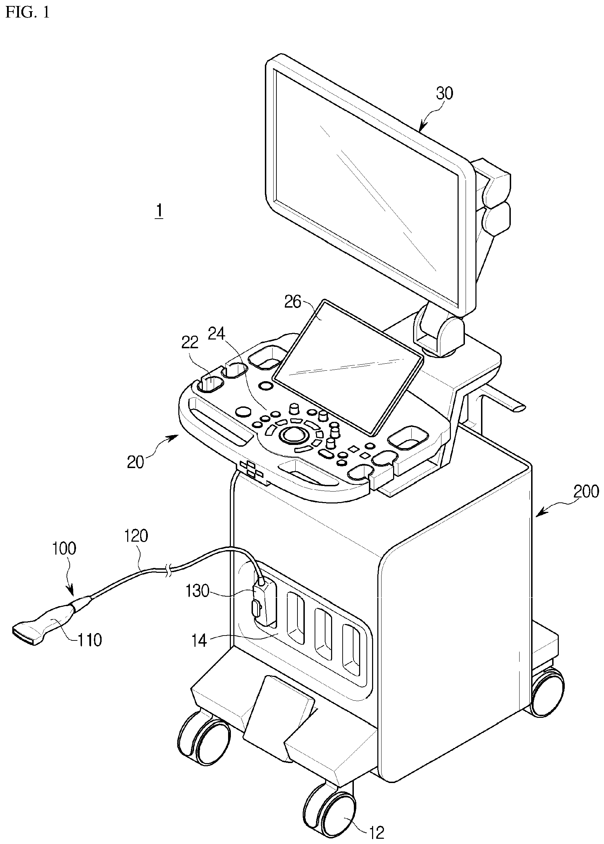

FIG. 1 is a view illustrating an appearance of an ultrasonic diagnostic apparatus according to an embodiment;
Referring to FIG. 1, the ultrasonic diagnostic apparatus according to an embodiment includes a main body 200 and an ultrasonic probe 100 that transmits an ultrasonic signal to an object to be diagnosed and receives a signal reflected from the object. The ultrasonic probe 100 may be connected to the main body 200 by a cable.

The ultrasonic probe 100 may be mounted on the main body 200 by a holder 22. When the user does not use the ultrasonic diagnostic apparatus 1, the ultrasonic probe 100 may be mounted on the holder 22 and stored. In FIG. 1, the holder 22 which mounts the ultrasonic probe 100 is shown as provided on the control panel 20, but it is also possible to be provided on the main body 200 at the user's convenience. In addition, it is also possible to be provided on both the main body 200 and the control panel 20.

The main body 200 may be provided with a moving device 12 to move the ultrasonic diagnostic apparatus 1. The moving device 12 may be a plurality of casters provided on the bottom surface of the main body 200. The caster may be aligned to move the main body 200 in a specific direction, or provided freely movable to be movable in any direction, or locked to be stopped at a specific position.

The ultrasonic probe 100 includes an ultrasonic transmission/reception device provided in the case 110. The ultrasonic reception device may include a transducer that irradiates ultrasound to an object, receives echo ultrasound reflected from the object, and converts the electrical signal and ultrasound to each other. Ultrasonic probe includes a male connector 130 that is physically coupled to a female connector 14 of the body 200 and transmits and receives signals to and from the body 200, and a cable 120 connecting the male connector 130 and the transducer.

Here, the object may be a human or animal living body, or an in vivo tissue such as a blood vessel, a bone, or a muscle, but is not limited thereto, and may be an object if its internal structure can be imaged by an ultrasonic diagnostic apparatus.

The echo ultrasound is ultrasound reflected from an object irradiated with ultrasound, and has various frequency bands or energy intensities for generating various ultrasonic images according to a diagnosis mode.

The transducer may generate ultrasonic waves according to the applied AC power. Specifically, the transducer may be supplied with AC power from an external power supply device or an internal power storage device, for example, a battery. The transducer vibrator may generate ultrasonic waves by vibrating according to the supplied AC power.

The cable 120 is connected to the transducer at one end and connected to the male connector 130 at the other end, thereby connecting the transducer to the male connector 130.The male connector 130 may be physically coupled to the female connector 14 of the body 200.The male connector 130 transmits the electrical signal generated by the transducer to the female connector 14 that is physically coupled, or receives the control signal generated by the body 200 from the female connector 14.

In FIG. 1, the male connector 130 and the cable 120 are shown exposed to the outside, but the male connector 130 and the cable 120 may be embedded in a housing forming the main body 200.

Meanwhile, a display 30 and a control panel 20 may be provided on the main body 200 of the ultrasonic diagnostic apparatus 1. The control panel 20 may be provided with an input 24 for the user to control the ultrasonic diagnostic apparatus 1. The input 24 can receive various control commands, as well as setting information about the ultrasonic probe 100 from the user.

According to an embodiment, the setting information regarding the ultrasonic probe 100 includes gain information, zoom information, focus information, time gain compensation (TGC) information, and depth information, Frequency information, power information, frame average information, and dynamic range information. However, the setting information regarding the ultrasonic probe 100 is not limited to one embodiment, and includes various information that can be set to take an ultrasonic image.

This information is transmitted to the ultrasonic probe 100 through a cable, and the ultrasonic probe 100 can be set according to the received information. In addition, the main body 200 may receive various control commands, such as a command for transmitting an ultrasonic signal, from the user through input 24, and transmit it to the ultrasonic probe 100.

Meanwhile, the input 24 may be implemented using a keyboard, a foot switch, or a foot pedal .For example, the keyboard can be implemented in hardware. Such a keyboard may include at least one of a switch, key, joystick and trackball. As another example, the keyboard may be implemented in software, such as a graphical user interface. In this case, the keyboard can be displayed through the display 30. A foot switch or a foot pedal may be provided below the main body 200, and a user may control the operation of the ultrasonic diagnostic apparatus 1 using the foot pedal.

The display 30 may be used in various known ways, such as a cathode ray tube (CRT), liquid crystal display (LCD), light emitting diode (LED), plasma display panel (PDP), organic light emitting diode (OLED), and the like. It may be implemented in various methods, but is not limited thereto.

The display 30 may display an ultrasonic image of a target portion inside the object. The ultrasonic image displayed on the display 30 may be a 2D ultrasonic image or a 3D ultrasound image, and various ultrasonic images may be displayed according to the operation mode of the ultrasonic diagnostic apparatus 1. In addition, the display 30 may display menus and instructions required for ultrasonic diagnosis, as well as information about the operating state of the ultrasonic probe 100.

Meanwhile, an auxiliary display 26 may be provided on the control panel 20.The secondary display 26 may provide related information such as a menu or a secondary image for optimizing the ultrasound image, or a graphic interface to the user.

In addition, when the auxiliary display 26 is implemented as a touch screen type, the display 30 may also perform the function of input 24. That is, the main body 200 may receive various commands from the user through at least one of the display 30 and the input 24. In addition, although not shown in the drawings, a voice recognition sensor is provided on the main body 200 to receive a voice command from a user.

Hereinafter, the configuration of the ultrasonic probe will be described in more detail.

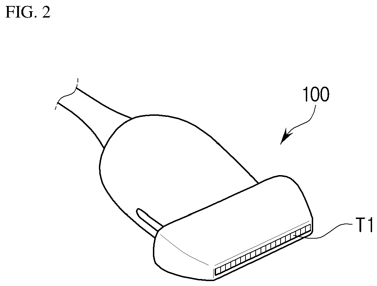

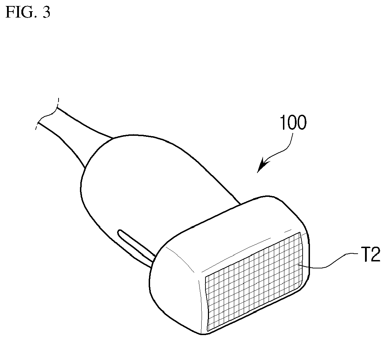

FIG. 2 is a view illustrating an appearance of an ultrasonic probe including a 1-Dimensional (ID) array transducer according to an embodiment; FIG. 3 is a view illustrating an appearance of the ultrasonic probe including a 2-Dimensional (2D) array transducer according to an embodiment; Hereinafter, descriptions will be made together to prevent duplication of descriptions.

The ultrasonic probe 100 is a part that contacts the surface of an object, and can irradiate an ultrasonic signal. Specifically, according to the control command signal received from the main body 200, the ultrasonic probe 100 irradiates the ultrasonic signal to the inside of the object, receives the echo ultrasonic signal reflected from a specific part inside the object, and transmits it to the main body 200. Accordingly, the ultrasonic probe 100 may transmit the echo ultrasound signal received from the object through the communication network to the main body 200 or acquire and transmit an ultrasound image from the echo ultrasound signal, and there is no limitation.

At this time, the ultrasonic probe 100 may include a transducer that mutually converts an electrical signal and an ultrasonic signal to transmit ultrasonic waves to the interior of the object. The transducer may be implemented as a one-dimensional or two-dimensional transducer array, and the transducer array is composed of a plurality of transducer elements.

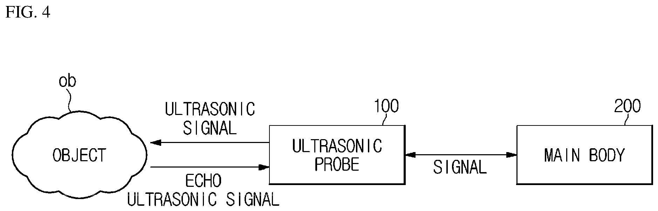

For example, the transducer may include a one-dimensional array transducer T1 as illustrated in FIG. 2. In another embodiment, the transducer may include a two-dimensional array transducer T2 as shown in FIG. 4.

For example, each transducer element constituting a one-dimensional array transducer may convert ultrasonic signals and electrical signals to each other. To this end, the transducer element may be implemented with a magnetostrictive ultrasonic transducer using a magnetostrictive effect of a magnetic body, Piezoelectric Ultrasonic Transducer using the piezoelectric effect of piezoelectric materials or piezoelectric micromachined ultrasonic transducer (pMUT), and it is also possible to implement a capacitive micromachined ultrasonic transducer (hereinafter abbreviated as cMUT) that transmits / receives ultrasonic waves using vibrations of hundreds or thousands of finely processed thin films.

Meanwhile, the transducers may be arranged in a linear shape, or may be arranged in a convex shape. In both cases, the basic operation principle of the ultrasonic probe 100 is the same, but in the case of the ultrasonic probe 100 in which the transducers are arranged in a curved surface, since the ultrasonic waves emitted from the transducers are in the shape of a fan, the generated ultrasonic image may also be in the shape of a fan.

Referring to FIG. 3, the transducer of the ultrasonic probe 100 may include a two-dimensional transducer array T2 as described above. When the 2D transducer array T2 is included, 3D imaging of the inside of the object may be performed.

Each of the transducer elements constituting the two-dimensional array transducer is the same as the transducer elements constituting the one-dimensional transducer array, and detailed description thereof will be omitted. Hereinafter, the relationship between the ultrasonic probe 100 and the main body 200 will be described.

FIG. 4 is a view illustrating a relationship between the ultrasonic probe 100 and a main body 200 according to an embodiment;

Referring to FIG. 4, the ultrasonic diagnostic apparatus 1 may include an ultrasonic probe 100 and a main body 200. The ultrasonic diagnostic apparatus (1) irradiates an ultrasonic signal from the surface of the object (ob) toward a target region in the body using the ultrasonic probe 100, and it is possible to obtain an image of a non-invasive way of a monolayer or blood flow of soft tissue by reflected ultrasonic signals, that is, echo ultrasonic signals.

For example, the ultrasonic probe 100 may irradiate a plane wave to the object ob through the two-dimensional transducer array T2.Here, the plane wave means an ultrasonic signal in the form of a 2D plane.

The ultrasonic probe 100 may receive an echo ultrasonic signal reflected from the object ob in response to irradiating ultrasonic waves to the object ob, and then transmit the received echo ultrasonic signal to the main body 200.

At this time, the main controller 200 may be provided with a main control unit that performs an image processing process for converting the received echo ultrasound signal into an ultrasound image. The main control unit may be implemented in the form of hardware such as a processor or a graphic processor, or alternatively, may be implemented in the form of software that can be executed on hardware. A detailed description of the main controller will be described later.

As another example, as described above, the ultrasonic probe 100 may directly convert the echo ultrasonic signal into an ultrasonic image and transmit it to the main body 200. The ultrasonic probe 100 is implemented in the form of hardware such as a processor or a graphic processor, or a probe controller implemented in the form of software that can be executed on hardware is provided to convert the echo ultrasonic signal into an ultrasonic image.

Meanwhile, the generated ultrasonic image may be stored in the memory 250 in the main body 200. In addition, the ultrasonic image may be stored in a web (Web storage) or cloud server that performs a storage function on the web.

The ultrasonic diagnostic apparatus 1 according to the disclosed embodiment aims to improve the workflow of an examiner and provide higher accuracy by acquiring a color Doppler image by detecting backflow of blood of a vein during ultrasonic examination and providing it for analysis.

To this end, the ultrasonic diagnostic apparatus 1 according to an embodiment includes an ultrasonic probe 100 to acquire a color Doppler image, a controller for determining the presence or absence of backflow of blood and the time of backflow based on the acquired color Doppler image, and a display that displays examination results. Here, determining the presence or absence of backflow of blood and the backflow time may be performed on the main controller of the main body as described above, or may be performed on the probe controller according to an embodiment. In the example described later, a case where a color Doppler image is analyzed by the probe controller and the main body controller controls the display unit based on the analyzed result will be described as an example, but the operation method of the present disclosure is not limited thereto.

Hereinafter, the internal configuration of the ultrasonic diagnostic apparatus 1 will be described in more detail.

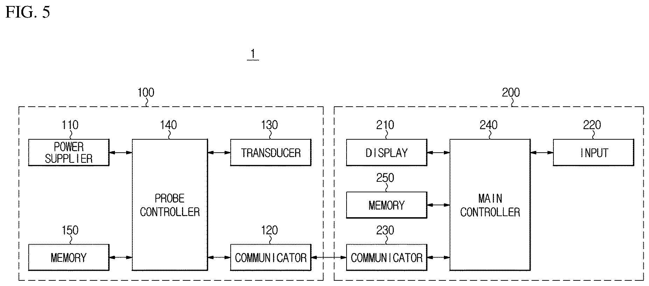

FIG. 5 is a view showing a control block diagram of an ultrasonic diagnostic apparatus 1 according to an embodiment.

Referring to FIG. 5, an ultrasonic diagnostic apparatus 1 according to an embodiment includes an ultrasonic probe 100 and a main body.

First, the configuration of the ultrasonic probe 100 will be described, and then the configuration of the main body 200 will be described.

The ultrasonic probe 100 includes a power supplier 110 that supplies power to the ultrasonic probe 100, a communicator 120 that transmits and receives various signals to and from external devices, and a transducer 130 that irradiates ultrasonic signals to an object and receives echo ultrasonic signals reflected from the object 130, a probe controller 140 for controlling the overall operation of the ultrasonic probe 100, and a memory 150 for storing various control data necessary for controlling the operation of the ultrasonic probe 100, echo ultrasonic signals, and the like.

Here, at least one of the communicator 120, the probe controller 140, and the memory 150 may be integrated in a system on chip (SOC) embedded in the ultrasonic probe 100 and operated by a processor. At this time, the ultrasonic probe 100 may not be provided with only one system-on-chip, and thus is not limited to being integrated on one system-on-chip.

The power supplier 110 can supply power to the ultrasonic probe 100. Specifically, the power supplier 110 converts and accumulates electrical energy into chemical energy, and then converts the accumulated chemical energy into electrical energy to supply power. According to an embodiment, the power supplier 110 may be implemented with a lithium ion battery, a nickel hydrogen battery, a polymer battery, or the like. However, the power supplier 110 is not limited to the above-described examples, and may be embodied in various types of batteries that are built into the ultrasonic probe 100 to supply power.

The power supplier 110 can be charged through a wired charging method that directly connects to a charging device or through a wireless charging method. That is, the charging method of the power supplier 110 may be performed according to various methods known in the art, and the charging method is not limited.

On the other hand, when the ultrasonic probe 100 is connected to the main body 200 through a wired communication method, the power supplier 110 may or may not be included in the ultrasonic probe 100 as required, but is not limited to that shown in FIG. 5.

Communicator 120 may include one or more components that enable wireless or wired communication with external devices. For example, communicator 120 may include at least one of a wireless communication module including at least one of a short-range communication module and a mobile communication module, and a wired communication module supporting a wired communication method.

The short-range communication module means a module for short-range communication within a predetermined distance. For example, short-range communication includes wireless LAN, Wi-Fi, Bluetooth, Zigbee, WFD (Wi-Fi Direct), UWB (Ultra wideband), infrared communication (IrDA; Infrared Data Association (BLE), Bluetooth Low Energy (BLE), NFC (Near Field Communication), and the like, but are not limited thereto.

The mobile communication module may transmit and receive a radio signal with at least one of a base station, an external terminal, and a server on a mobile communication network. For example, the mobile communication module may transmit and receive various types of data to and from the main body 200 via a base station through a 3G or 4G communication network. Hereinafter, the short-range communication module and the mobile communication module will be referred to as communication modules.

The wired communication module means a module that supports sending and receiving signals containing data over a wire. For example, the wired communication module may support at least one of various known wired communication methods such as Peripheral Component Interconnect (PCI), PCI-express, and Universe Serial Bus (USB).

The communicator 120 can transmit and receive various data with the main body 200 through a communication network. The communicator 120 may transmit and receive data related to the diagnosis of the object, such as ultrasound images, echo ultrasound data, and Doppler data about the object through a communication network. Also, the communicator 120 may receive various control command signals from the main body 200. That is, there is no limit to the type of data or commands that communicator 120 can send and receive via wired/wireless signals.

Meanwhile, the transducer 130 may be provided in the ultrasonic probe 100 as described above. The transducer 130 may irradiate an ultrasound signal to an object and receive an echo ultrasound signal reflected from the object. Since the description of the transducer 130 has been described above, a description of the same content will be omitted.

The probe controller 140 may control the overall operation of the ultrasonic probe 100. The probe controller 140 may be implemented through at least one of a processor capable of performing various processes such as an image processing process, an operation processing process, and a graphics processor, or implemented through components integrated with the functions of the aforementioned processors.

The probe controller 140 may generate a control signal according to a user's control command, and control the overall operation of the components of the ultrasonic probe 100 through the generated control signal. For example, control data for controlling the components of the ultrasonic probe 100 and control data for performing an image processing process may be stored in the memory 150 in advance. The probe controller 140 may generate a control signal based on data stored in the memory 150 and control the overall operation of the components of the ultrasonic probe 100 through the generated control signal.

The probe controller 140 may acquire an ultrasonic image from the echo ultrasonic signal received by the transducer 130. For example, the probe controller 140 may acquire an ultrasonic image by performing an image processing process based on control data stored in the memory 150 for the echo ultrasonic signal received through the transducer 130.

The ultrasound image may include a gray scale image obtained by scanning an object according to any one of A-mode (Amplitude mode), B-mode (Brightness mode), and M-mode (Motion mode).

Also, ultrasonic images includes a color Doppler image expressing the movement of the object using the Doppler effect (Doppler effect) depending on the C-mode (Color Doppler mode) may include a color Doppler image expressing the movement of the object using the Doppler effect (doppler effect), and a spectral Dopppler image providing a Doppler spectrum depending on the D-mode,.

The disclosed embodiment relates to detecting the presence or absence of backflow of blood based on a color Doppler image and automatically positioning a sample volume of a pulse wave, hereinafter, embodiments will be described on the assumption that the C and D-modes are set.

The probe controller 140 may generate a color Doppler image from the received echo ultrasonic signal, and calculate the presence or absence of backflow of blood and the time of backflow in the blood vessel based on the color Doppler image. Here, the backflow time of blood is obtained based on a time point after squeezing and releasing the subject's body part, and the backflow of blood is caused by a method of applying pressure to the subject's body part or by a Valsalva's maneuver. Here, the method of applying pressure to the subject's body part may be a method of using a cuff and pressing with a hand. However, the method of causing backflow of blood is not limited by the above-described examples, and it is needless to say that the method of causing backflow of blood widely used in the art may be used.

The probe controller 140 may control the communicator 120 to transmit a color Doppler image to the main body 200, the main controller 240 of the main body 200 may control the display unit of the main body 200 to display the presence or absence of backflow of blood and the time of occurrence of backflow based on the received color Doppler image. As a result, the examiner can check the presence or absence and backflow time of blood in the blood vessel by checking the color Doppler image through the display unit of the main body 200.

The probe controller 140 may automatically set the sample volume of the pulse wave based on the acquired color Doppler image, and analyze the spectrum of the blood flow at the set sample volume to calculate blood flow velocity, blood flow velocity for each direction in more detail, and the like. Here, the blood flow velocity may include an average velocity, a maximum velocity, and a minimum velocity of the blood flow.

The method of automatically setting the sample volume of the pulse wave based on the color Doppler image in the probe controller 140 may include a method of randomly setting the sample volume, the method may be a method of setting a sample volume in a central portion of a color Doppler image, but is not limited thereto.

The probe controller 140 may control the communicator 120 to transmit blood flow spectrum analysis results to the main body 200, and the main controller 240 of the main body 200 may display the received analysis result to display the main body 200 210 can be controlled.As a result, the examiner can check the blood flow spectrum together with the color Doppler image through the display unit 210 of the main body 200 to check the presence or absence of backflow of blood in the blood vessel, backflow time, blood flow rate, blood flow velocity, and blood flow direction.

The probe controller 140 may control the communicator 120 to transmit the blood flow spectrum analysis results to the main body 200,The main controller 240 of the main body 200 may control the display 210 of the main body 200 to display the received analysis result. As a result, the examiner can confirm the presence or absence of backflow of blood, backflow time, blood flow rate, blood flow velocity, and blood flow direction in the blood vessel by checking the blood flow spectrum together with the color Doppler image through the display 210 of the main body 200.

The memory 150 may control components of the ultrasonic probe 100 and store a control program, application, or data required to perform an imaging process for acquiring ultrasonic images.

In more detail, the memory 150 may acquire a color Doppler image based on the echo ultrasonic signal, and store a control program, application, or control data for determining the presence or absence and backflow time of blood based on the acquired color Doppler image. In addition, the memory 150 automatically set the sample volume of the pulse wave to the color Doppler image based on the color Doppler image, and stores application or control data for calculating a blood flow velocity including at least one of an average velocity, a maximum velocity, and a minimum velocity by analyzing the blood flow spectrum of the sample volume based on the blood flow information obtained from the ultrasonic probe 100A control program.

The memory 150 can be implemented through a storage medium of at least one type of a flash memory type, a hard disk type, a multimedia card micro type, a card type memory (for example, SD or XD memory, etc.), Random Access Memory (RAM), Static Random Access Memory (SRAM), Read-Only Memory (ROM), Electrically Erasable Programmable Read-Only Memory (EPMROM), Programmable Read-Only Memory (PROM), magnetic memory, a magnetic disk or an optical disk. However, it is not limited thereto, and may be implemented in any other form known in the art.

Meanwhile, the components of the ultrasonic probe 100 are not limited to the above-described examples. For example, the ultrasonic probe 100 may be further provided with a display if necessary. Information related to an operating state of the ultrasonic probe 100 may be displayed on the display unit, such as a power state of the ultrasonic probe 100.

In addition, an input may be further provided in the ultrasonic probe 100 as necessary. The input may receive power on and off commands of the ultrasonic probe 100 from a user. The input may be provided to receive an execution command for a color Doppler mode from a user in order to analyze the backflow of blood in the blood vessel, and may also be provided to receive a control command for changing the operation mode of the ultrasonic probe 100. The input may be implemented in the form of a switch, a key, and the like, and is not limited.

Next, the main body 200 may include a display 210, an input 220, a communicator 230, a main controller 240 and a memory 250. Here, at least one of the communicator 230, the main controller 240, and the memory 250 may be integrated in a system-on-chip embedded in the main body 200 and operated by a processor.

At this time, since there is not only one system-on-chip embedded in the main body 200, it is not limited to being integrated into one system-on-chip .On the other hand, the description of the input 220 and communicator 230 has been described above, so the same description will be omitted.

The display 210 can display various information including an ultrasonic image obtained through an image processing process from an echo ultrasound signal. In more detail, display 210 may display a test result screen together with a color Doppler image.

For example, display 210 displays a color Doppler mode entry screen as the inspection process progresses, displays the first test result screen including information about the presence or absence of backflow of blood and time of backflow, displays the sample volume setting screen, displays a second test result screen indicating a blood flow analysis result of the sample volume. An example of a screen displayed on the display 210 according to the progress of the inspection process will be described later in a relevant part.

When the display 210 is implemented as a touch screen type, the display 210 is displayed with a graphical user interface (GUI), so that a user can receive various control commands for the ultrasonic probe 100 as well as the main body 200. Accordingly, the display 210 implemented as a touch screen type may perform a function of an input.

The communicator 230 can exchange data with external devices through a communication network. Here, the communication network includes a wired/wireless communication network. The detailed description of the wired/wireless communication network is the same as described above, so it will be omitted.

The communicator 230 can exchange various signals with the ultrasonic probe 100 through a communication network as described above. Referring to FIG. 5, the ultrasonic probe 100 and the main body 200 are connected via communicator 120 of the ultrasonic probe 100 and communicator 230 of the main body 200 to exchange and receive various data. For example, communicator 230 may transmit various control commands, and may receive an echo ultrasound signal or a signal including ultrasound image data reconstructed from the echo ultrasound signal.

The memory 250 may store control programs, applications, or data for controlling components of the main body 200.

Regarding the information that can be stored in the memory 250 of the main body, the content overlapping with the description of the memory 150 of the ultrasonic probe 100 described above will be omitted.

Regarding the information that can be stored in the memory 250 of the main body, the content overlapping with the description of the memory 150 of the ultrasonic probe 100 described above will be omitted.

Meanwhile, the memory 250 may store a program (hereinafter referred to as a guide program) capable of guiding a patient's diagnosis. In order to perform an intravenous vein examination through the ultrasound diagnostic apparatus according to the disclosed embodiment, an examination of a plurality of test sites must be performed. In this case, the guide program stored in the memory 250 may guide the inspection order for a plurality of inspection sites through the display 210 in order to facilitate the inspection work of the examiner.

For example, the guide program guides the inspection for the first position so that the inspection for the first position proceeds, and when the inspection for the first position is completed, the guide program may control the display 210 so that the inspection result for the first position is automatically reported. Subsequently, the guide program guides the inspection for the second position so that the inspection for the second position proceeds according to the programmed inspection order, and when the inspection for the second position is completed, the inspection result for the second position is automatically reported. In addition, the guide program can display the ultrasonic image of the site being diagnosed by the examiner on the display 210 while guiding the aforementioned procedure. In addition, the guide program can guide by displaying an ultrasound image mode, annotation, display layout, image parameter, and probe preset.

In addition, the memory 250 may store patient information obtained using the ultrasonic probe 100 during each inspection step. The patient's information may include the ultrasound image acquired by the ultrasonic probe 100 and the set value of the ultrasonic probe 100 set to acquire the ultrasound image.

The main controller 240 controls the overall operation of the ultrasonic diagnostic apparatus 1 and the signal flow between the internal components of the ultrasonic diagnostic apparatus 1 and processes data.

The main controller 240 may be implemented through at least one of a processor capable of performing various processes such as an image processing process, an arithmetic processing process, and a graphic processor, or through a single integrate component has the functions of the aforementioned processors.

The main controller 240 may generate a control signal according to a user's control command, and control the overall operation of the ultrasonic diagnostic apparatus through the generated control signal. For example, in the main controller 240, the same process as the image processing process and the calculation processing process performed in the above-described probe controller 140 may be performed. In other words, the main controller 240 may calculate the presence or absence of backflow of blood and the time of backflow in the blood vessel based on the color Doppler image described above, and control the display 210 of the main body 200 to display the calculated result.

When the main controller 240 is tested based on the guide program stored in the memory 250, the main controller 240 provides a guide screen for guiding the inspection for the first position so that the inspection for the first position proceeds, and when the inspection for the first position is completed, the display of the display 210 can be controlled so that the inspection result for the first position is automatically reported. Subsequently, the main controller 240 provides a guide screen for guiding the inspection for the second position so that the inspection for the second position proceeds according to the inspection sequence programmed in the guide program, and when the inspection for the second position is completed, the display of the display 210 can be controlled so that the inspection result for the second position is automatically reported.

Hereinafter, descriptions overlapping with those described in the probe controller 140 described above in relation to the main controller 240 will be omitted.

The control configuration of the ultrasonic diagnostic apparatus 1 according to the disclosed embodiment has been described above.

Next, it will be described with respect to the control process of the ultrasonic diagnostic apparatus 1 according to the disclosed embodiment on the premise of the configuration of the ultrasonic diagnostic apparatus 1 described above.

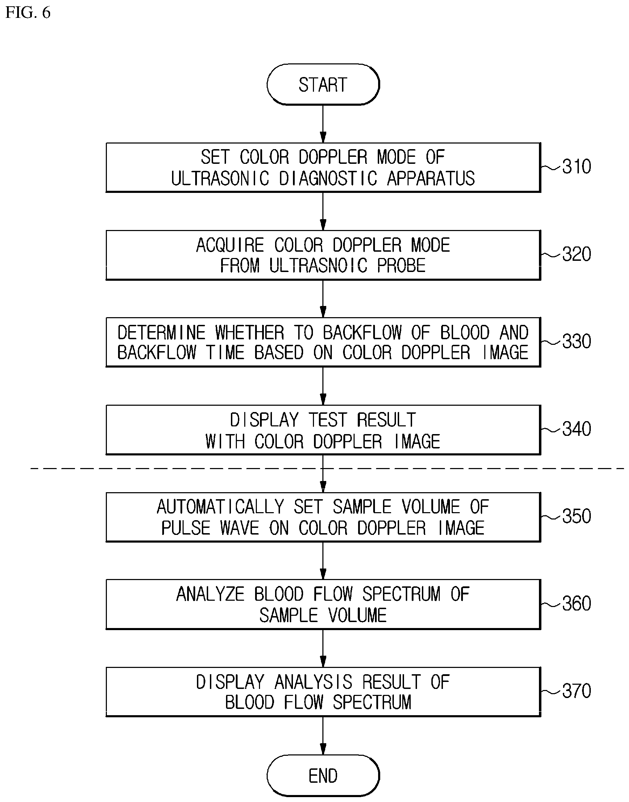

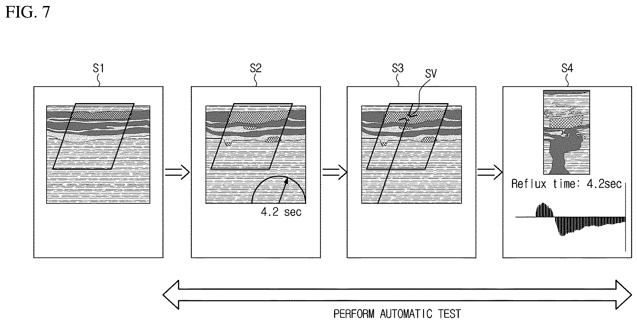

FIG. 6 is a flowchart illustrating a control method of the ultrasonic diagnostic apparatus 1 according to an embodiment; FIG. 7 is a diagram illustrating a workflow according to the control process of FIG. 6.

Referring to FIGS. 6 and 7, Control method of the ultrasonic diagnostic apparatus 1 according to an embodiment includes Setting the color Doppler mode of the ultrasonic diagnostic apparatus 1 (310), acquiring a color Doppler image from the ultrasonic probe 100 (320) based on the color Doppler image, determining whether there is backflow of blood and backflow time (330), and displaying the inspection result together with the color Doppler image (340).

According to an embodiment, the control method of the ultrasonic diagnostic apparatus 1 follows the above steps includes automatically setting a sample volume of the pulse wave in the color Doppler image based on the color Doppler image (350); analyzing the blood flow spectrum of the sample volume based on the blood flow information obtained from the ultrasonic probe 100 (360), and displaying a result of analyzing the blood flow spectrum (370).

First, the step of setting the color Doppler mode of the ultrasonic diagnostic apparatus 1 is performed. The tester may input an execution command for the color Doppler mode through the input unit provided in the ultrasonic probe 100 or the input 220 provided in the main body 200, and set a color box (CB) in the B-mode image. The ultrasonic diagnostic apparatus may control the ultrasonic diagnostic apparatus 1 to operate in the color Doppler mode based on an execution command for the color Doppler mode received from the input.

When the ultrasonic diagnostic apparatus 1 operates in a color Doppler mode, the ultrasonic probe 100 forms a color Doppler image based on the received echo ultrasonic signal. In more detail, the ultrasonic probe 100 may acquire a Doppler signal corresponding to a color box (CB) set by the user and form a color Doppler image based on the obtained Doppler signal.

The first picture of FIG. 7 shows an example of the color Doppler mode entry screen S 1 provided on the display 210 of the main body 200 when the ultrasonic diagnostic apparatus 1 enters the color Doppler mode.

When ultrasonic diagnostic apparatus 1 enters the color Doppler mode and performs squeezing and release to the subject, blood backflow occurs in the blood vessel of the subject, and the ultrasonic probe 100 may acquire a Doppler signal generated by backflow of blood and form a color Doppler image based on the obtained Doppler signal.

Referring to the second picture of FIG. 7, when squeezing and releasing to an examiner, it can be confirmed that a Doppler image of a color different from the first picture of FIG. 7 is provided on the display 210 of the main body 200. The examiner can intuitively check the presence or absence of blood backflow of the examinee based on the color change of the color Doppler image.

The ultrasonic diagnostic apparatus 1 may determine the presence or absence of backflow of blood and the time of backflow based on the color Doppler image, and display the test results together with the color Doppler image on the display 210 of the main body 200. Hereinafter, a screen displaying test results for the presence and absence of backflow of blood and the backflow time is defined as a first test result screen (S2).

Referring to the second picture of FIG. 7, a graphic or text for visually providing a test result together with a color Doppler image may be provided on the first test result screen S2. In more detail, one end of the color Doppler image may be provided with a graphic or text to indicate the backflow time of blood. The examiner can determine the presence or absence of backflow of blood based on the color provided through the color Doppler image, and the presence or absence of backflow of blood and the backflow time of the blood can be confirmed through graphics or text provided at one end of the color Doppler image..

Next, a step of automatically setting the sample volume of the pulse wave in the color Doppler image based on the color Doppler image may be performed. Such a process may be automatically performed after providing the first test result screen S2 according to the setting of the examiner, thereby simplifying the examiner's inspection process.

For example, referring to FIG. 7, a sample volume setting screen (S3) for automatically setting a sample volume (SV) of a pulse wave in a color Doppler image is provided after a predetermined time has elapsed after providing the first inspection result screen. The method of automatically setting the sample volume (SV) of the pulse wave may include a method of randomly setting the sample volume, and may be a method of setting the sample volume in the center portion of the color Doppler image according to an embodiment, but it is not limited thereto.

Next, based on the blood flow information obtained from the ultrasonic probe 100, a step of analyzing the blood flow spectrum of the sample volume and a step of displaying the analysis result of the blood flow spectrum may be performed.

The probe controller 140 may analyze the blood flow spectrum of the sample volume based on the blood flow information obtained from the ultrasonic probe 100, and display the blood flow spectrum analysis result of the sample volume through the display 210 of the main body 200 by transferring the analysis result to the main controller 240 of the main body 200. Hereinafter, a screen displaying the analysis result of the blood flow spectrum of the sample volume is defined as a second test result screen S4.

For example, referring to FIG. 7, a blood flow spectrum for visually providing test results together with a color Doppler image may be provided on the second test result screen S4. The blood flow spectrum may be provided in the form of graphics and text, including blood flow velocity and blood flow direction information, and the user can check the speed of the blood flow, for example, the maximum speed, the average speed, and the minimum speed and direction of the blood flow through the blood flow spectrum.

In the above, the control process of the ultrasonic diagnostic apparatus 1 according to an embodiment has been described. In the control process of the ultrasonic diagnostic apparatus 1In the first and second test result screens described above, a diagnosis grade for backflow time may be provided along with information on whether or not there is backflow of blood, backflow time, and backflow rate, and a body marker may be provided to provide information on a body part of an examinee who has undergone the examination and an analysis result for the corresponding region according to an embodiment,

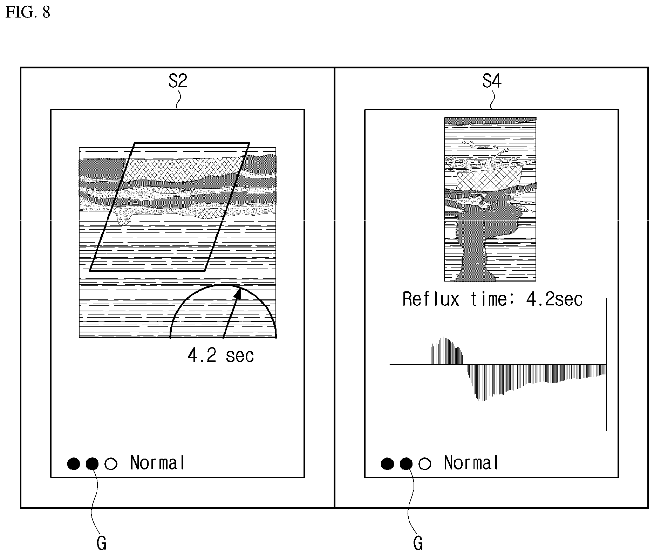

FIG. 8 is a diagram illustrating an example in which the diagnostic grade for the time of backflow is displayed on the first and second test result display screens.

The diagnostic grades for the detected backflow time may be stored in the memories 150 and 250 of the ultrasonic diagnostic apparatus 1 in advance. For example, the range of the detected backflow time can be classified step by step, and the diagnostic grade can be classified into low risk, normal, and risk grade according to the range. The range of diagnostic grades can be classified according to the designer's intention.

Referring to FIG. 8, graphic or text information indicating a diagnostic grade for the backflow time described above may be displayed on one area of the first and second test result display screens. The examiner may more intuitively check the state of the examinee based on graphic or text information provided on the screen.

Referring to FIG. 8, graphic or text information indicating a diagnostic grade for the backflow time described above may be displayed on one area of the first and second test result display screens. The examiner may more intuitively check the state of the examinee based on graphic or text information provided on the screen.

FIG. 8 shows a step-by-step display of the diagnostic grade for the backflow time with a plurality of indicators, and a text for a diagnostic grade is provided around the indicator, the example of providing the diagnostic grade is not limited by that shown in FIG. 8. In other words, the diagnostic grade may be provided in a form in which the color of the indicator is changed according to the grade or may be provided only in text.

In addition, the diagnostic grade may be displayed on at least one of the first test screen and the second test screen, and the color Doppler mode entry screen and the sample volume setting screen may also be displayed depending on the embodiment.

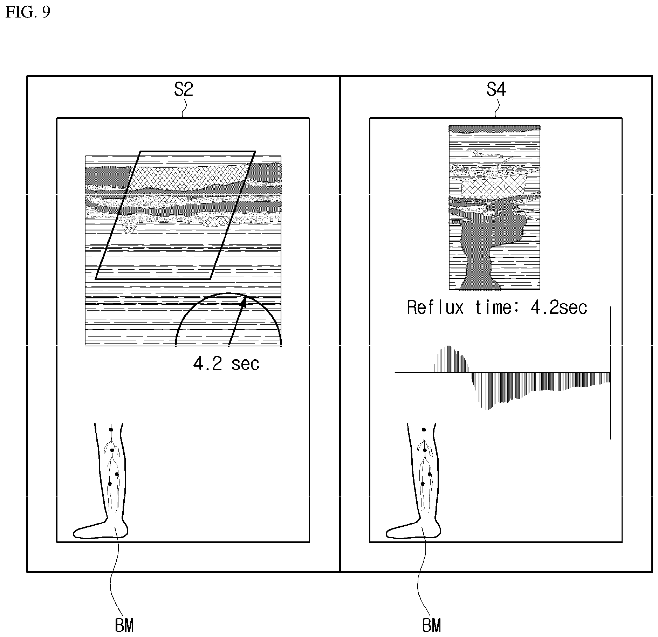

FIG. 9 is a diagram illustrating an example in which a body marker is displayed on the first and second test result display screens. The body marker is provided to visually confirm the test site of the examinee, and the body marker may be provided in a form that can easily identify a portion of the target test site where the test is completed. For example, the body marker may be provided in the form of a graphic image of the inspected portion of the examinee, and mark the portion where the examination is completed, so that the examiner can perform the examination more conveniently. FIG. 9 shows an example of a displayable body marker, and examples of the displayable markers on the first and second inspection screens are not limited to those illustrated in FIG. 9.

In addition, the body marker may be displayed on at least one of the first test screen and the second test screen, and of course, it may be displayed on the color Doppler mode entry screen and the sample volume setting screen according to an embodiment.

The control method of the ultrasonic diagnostic apparatus 1 and ultrasonic diagnostic apparatus 1 according to an embodiment has been described above. The technical spirit of the invention is not limited to the above-described embodiments, and should be broadly understood as a concept including changes within a range that can be easily thought by those skilled in the art.

## Claims

1. An ultrasonic diagnostic apparatus comprising:
an ultrasonic probe configured to acquire a color Doppler image;
a controller configured to determine a presence or absence of backflow of blood and a time of backflow based on the acquired color Doppler image; and
a display configured to display an examination result with the color Doppler image.

2. The ultrasonic diagnostic apparatus of claim 1, wherein the examination result includes at least one of the presence or absence of backflow of blood and the time of backflow.

3. The ultrasonic diagnostic apparatus of claim 1, wherein the controller automatically sets a sample volume of a pulse wave in the color Doppler image based on the color Doppler image, and analyzes a blood flow spectrum of the sample volume based on a blood flow information acquired from the ultrasonic probe.

4. The ultrasonic diagnostic apparatus of claim 3, wherein the controller calculates a blood flow velocity including at least one of an average velocity, a maximum velocity and a minimum velocity by analyzing the blow flow spectrum of the sample volume.

5. The ultrasonic diagnostic apparatus of claim 3, wherein the display displays a result of the blood flow spectrum analysis of the sample volume.

6. The ultrasonic diagnostic apparatus of claim 3, wherein the result of the blood flow spectrum analysis includes at least one of the presence or absence of backflow of blood, the time of backflow, amount of blood flow, blood flow rate, and blood flow direction.

7. The ultrasonic diagnostic apparatus of claim 1, wherein the controller determines a diagnostic grade for the time of backflow, and
wherein the display displays the determined diagnostic grade.

8. The ultrasonic diagnostic apparatus of claim 1 further comprising:
an input configured to receive an execution command for a color Doppler mode from a user in order to analyze the backflow of blood.

9. The ultrasonic diagnostic apparatus of claim 1, wherein the display displays information about a subject's body parts that have been observed and results of an analysis on the subject's body parts.

10. The ultrasonic diagnostic apparatus of claim 1, wherein the time of blood backflow obtains based on the time of release after squeezing the body part of the subject.

11. A control method of an ultrasonic diagnostic apparatus comprising:
acquiring a color Dopplier image from an ultrasonic probe;
determining a presence or absence of backflow of blood and a time of backflow based on the acquired color Doppler image; and
displaying an examination result with the color Doppler image.

12. The method of claim 11 further comprising:
automatically setting a sample volume of a pulse wave in the color Doppler image based on the color Doppler image;
analyzing a blood flow spectrum of the sample volume based on a blood flow information acquired from the ultrasonic probe; and
displaying a result of the blood flow spectrum analysis.

13. The method of claim 12, wherein
analyzing the blood flow spectrum comprises:
calculating a blood flow velocity including at least one of an average velocity, a maximum velocity and a minimum velocity by analyzing the blow flow spectrum of the sample volume.

14. The method of claim 11 further comprising:
determining a diagnostic grade for the time of backflow, and
displaying the determined diagnostic grade.

15. The method of claim 11, wherein the time of blood backflow includes the time of blood backflow obtained based on the time of release after squeezing the body part of the subject.
